Europäisches Patentamt

(19) European Patent Office

Office européen des brevets

(11) **EP 0 931 142 B1**

(12) **EUROPEAN PATENT SPECIFICATION**

(45) Date of publication and mention
of the grant of the patent:
**07.05.2003 Bulletin 2003/19**

(51) Int Cl.[7]: **C12N 11/04**, C12P 7/46

(21) Application number: **97942129.4**

(22) Date of filing: **05.09.1997**

(86) International application number:
**PCT/HU97/00047**

(87) International publication number:
**WO 98/010061 (12.03.1998 Gazette 1998/10)**

(54) **METHOD AND IMMOBILIZED BIOCATALYST FOR THE PRODUCTION OF L(-) MALIC ACID**

VERFAHREN UND IMMOBILISIERTER BIOKATALYSATOR ZUR HERSTELLUNG VON L(-)
APFELSÄURE

PROCEDE ET BIOCATALYSEUR IMMOBILISE SERVANT A LA PRODUCTION D'ACIDE
L(-)MALIQUE

(84) Designated Contracting States:
**AT DE FR GB IT**
Designated Extension States:
**LT**

(30) Priority: **05.09.1996 HU 9602431**

(43) Date of publication of application:
**28.07.1999 Bulletin 1999/30**

(73) Proprietor: **Bay Zoltan Alkalmazott Kutatasi
Alapitvany
1116 Budapest (HU)**

(72) Inventors:
• **POLYAK, Béla**
  **H-6723 Szeged (HU)**
• **KALMAN, Miklos**
  **H-6723 Szeged (HU)**
• **KISS-TOTH, Endre**
  **H-6722 Deszk (HU)**
• **DORGAI, Lászlo**
  **H-6726 Szeged (HU)**
• **TOTH, Mária**
  **H-6723 Szeged (HU)**
• **HANSEL, Miklos**
  **H-6724 Szeged (HU)**
• **BENJAMIN, Arpád**
  **H-6723 Szeged (HU)**

(74) Representative: **Viering, Jentschura & Partner
Postfach 22 14 43
80504 München (DE)**

(56) References cited:
**WO-A-93/24112**          **US-A- 4 352 883**

• **DATABASE WPI Week 9744 Derwent
  Publications Ltd., London, GB; AN 97-474060
  XP002052928 & HU 72 927 A (BAY ZOLTAN
  ALKALMAZOTT KUTATASI ALAPITVA), 28 June
  1996 cited in the application**
• **SMIDSROD O ET AL: "ALGINATE AS
  IMMOBILIZATION MATRIX FOR CELLS"
  TRENDS IN BIOTECHNOLOGY, vol. 8, no. 3, 1
  March 1990, pages 71-78, XP000095653**
• **DATABASE BIOSIS BIOSCIENCES
  INFORMATION SERVICE, PHILADELPHIA, PA,
  US ABSTRACT NO. 95:106627, WAKAO ET AL:
  "IMMOBILIZATION OF THIOBACILLUS
  FERROOXIDANS USING VARIOUS POLYMERS
  AS MATRIX" XP002052927 & JOURNAL OF
  GENERAL AND APPLIED MICROBIOLOGY, vol.
  40, no. 4, 1994, pages 349-358,**

Note: Within nine months from the publication of the mention of the grant of the European patent, any person may give
notice to the European Patent Office of opposition to the European patent granted. Notice of opposition shall be filed in
a written reasoned statement. It shall not be deemed to have been filed until the opposition fee has been paid. (Art.
99(1) European Patent Convention).

**Description**

The present invention relates to a novel continuous production process for the preparation of L(-) malic acid (the naturally occurring enantiomer of malic acid) from fumaric acid with the use of a novel immobilized biocatalyst. The long shelf life novel immobilized biocatalyst comprising living or killed cells or purified enzymes suitable for use in the method of the invention is also comprised in the invention.

The method and immobilized biocatalyst of the present invention is useful for cost effective stereoselective industrial scale preparation of natural malic acid.

With respect to the present specification and claims, the foregoing technical terms will be used in accordance with the below given definitions. With regard to the interpretation of the present invention, it shall be understood that the below defined terms are used in accordance with the given definitions even if said definitions might not be in perfect harmony with the usual interpretation of said technical term in the art.

The term *"molecular composite gel"* is used to define gels made of macromolecules wherein more than one type of macromolecules participates in the formation of the gel structure.

A "*membrane impermeable for globular proteins of a given size"* is a membrane through which globular proteins of a size equal to or larger than the given size (defined in molecular mass units) can not penetrate by non facilitated diffusion within 10 hours in room temperature in more than 1% quantity.

With regard to the present specification gels composed of linear polymers of sugar units (or modified sugar units) of 6 carbon atoms connected via $\alpha$-1-4 bonds and having defined melting and gelling points are called "g*els of agarose related structure".*

With regard to the present invention the terms *"malic acid"* and *"fumaric acid"* are used with a broadened meaning also including the salts of the said acids present under physiological circumstances (and also when practicing the method of the invention). In the above meaning the said expressions are also used as synonyms of the terms *"malate"* and *"fumarate",* respectively (as it is also common in the art). Accordingly, an e.g. 1 M solution of *"malic acid"* buffered to a certain pH value is in fact one molar for malate ions (dissociated or not) and not for malic acid.

The natural form of malic acid is present in every living cell as an element of one of the main energetic systems of the cells, the so called Szent-Györgyi-Krebs cycle. The direction of L-malate transformation in the cycle is as follows:

$$\text{L-malate} \rightarrow \text{fumarate} + H_2O.$$

This reaction is catalyzed in the cells by the fumarase enzyme (E.C.4.2.1.2) . The equilibrium constant of the above reaction is 0.23. The enzyme catalyzing the reaction is absolutely specific for both fumaric acid and L-malic acid. The natural regulatory inhibitors of the enzyme are succinate ($K_i$=5.2x10$^{-2}$) and citric acid ($K_i$=5.5x10$^{-3}$).

The equilibrium set by fumarase in the living cells ensures the biotechnological applicability of the enzyme. If the concentration of fumaric acid is elevated in the environment of living cells, the system endeavors towards equilibrium. The direction of the reaction catalyzed by the enzyme is, thus, reversed and natural malic acid is produced in high quantities.

The above reaction is fully stereoselective unlike the chemical synthesis starting from fumaric acid or maleic anhydride resulting in racemic malic acid. The fumarase enzyme is continuously transcribed and the enzyme is synthesized through the whole life cycle of the cell. The enzyme, however, is also inducible meaning that it's copy number can be increased in the cell by adding fumaric acid, malonic acid, tartarate or competitive inhibitors of the enzyme to the culture medium. The level of fumarase, however, is not equal in all different cell types. This fact, in technological sense, limits the number applicable cell types.

Alkali salts of both fumaric acid and malic acid are well soluble in water and they are capable of penetrating through biological membranes.

The applicability of L(-) malic acid is extremely widespread. It is produced in a quantity of about 50,000 ton/year worldwide. Because of the presently accelerating needs in the market, worldwide production of malic acid will probably be multiplied very soon.

Besides the significant role malic acid plays in the energy production of the living cells, it is also important physiologically in storing and delivering different metal ions in complexed forms within the living organisms. This biological function of malic acid provides the basis for the application of its calcium salt for the prophylaxis and treatment of old age associated osteoporosis.

Being an excellent chelator, malic acid also serves as an essential ingredient of optimized micro-element preparations used in human prophylaxis and therapy.

Different roborants are comprising mixtures of amino acids present in the form of (L) malic acidic salts.

It is well known in the art that during the maturation process of different meats lactic acid is formed and also accumulates. An industrial process has recently been started to use in the USA according to which natural malic acid is used to accelerate the maturation process of different types of meats and also for preservation. If this method becomes more extensively used (as it is expected), the market need for malic acid will, of course, significantly grow worldwide.

Malic acid is also used by the cosmetic industry. It is mainly employed in the so called fruity acid containing face and body lotions. It can be noted that the mechanism of action of such preparations is based on the induction of the Krebs cycle in the skin cells.

Malic acid is also important in soft drink production where it can replace citric acid. Usage of malic acid instead of citric acid results - for obvious biochemical reasons - in less fattening products, as malic acid exits the Krebs cycle by activating cell respiration, while citric acid induces fatty acid synthesis (as it's decomposition results in the formation of acetyl-CoA).

Chemical industry is also expected to use increasing amount of natural malic acid as biodegradable polymers can be produced from it.

The usefulness of the fumarate → malate reaction is recognized by many authors and a vast number of technologies for the production of L(-) malic acid has been elaborated.

Production of malic acid in the organic chemical industry is a simple, well established method which is based on the hydrolysis of maleic anhydride. The product of the reaction is an about 50-50% mixture of fumaric and malic acids. Fumaric acid can be removed from the reaction mixture by simple acidification step. After isolating fumaric acid, the mother liquor will comprise racemic (D-L) malic acid. Resolving this racemic mixture for producing optically homogeneous L(-) malic acid, however, is rather complicated.

Fumarase enzyme present in the living organisms, however, is very suitable for producing stereospecifically L(-) malic acid from fumaric acid. A biotechnological method for producing D(+) malic acid - based on the selective decomposition of the natural isomer - has also been disclosed.

Shigeo Abe et al. was granted a patent in 1960 for their fermentation process for the production of L(-) malic acid (US 3,063,910). The authors used different molds for the preparation of malic acid, e.g. *Apergillus parasiticus, A. flavus* and *A. orysae.* These molds can produce malic acid from different carbon sources and the product accumulates in the culture supernatant. The authors used glucose, fructose, mannose, xilose, starch and glycerol as carbon source. $CaCO_3$ has also been added to the fermentation medium. It was established that certain cations (like $Al^{3+}$ and $Cr^{3+}$) favorably influenced malic acid production during the fermentation process. A production cycle lasted 5-9 days and malic acid was produced in a quantity of 50 mg/ml.

Fritz Schindler (DE 3,310,849; 1983) has produced L(-) malic acid in very high concentration (100-170 g/l) from fumaric acid and using molds, too. Species employed were as follows: *Aspergillus ventii, Helmintosporium sativum and Penicillium nalgiovensys.* His method was a batch type fermentation, starting from a neutralized 12.5% solution of fumaric acid supplemented with the necessary nutrient components. The whole fermentation process lasted 3 days. The conversion rate was around 70%.

The elegant procedure of Kitahara Kano (1959) is essentially based on the higher solubility of the calcium salt of fumaric acid compared to that of the calcium salt of L(-) malic acid. When adding solid fumaric acid and $CaCO_3$ into the fermentor, calcium-fumarate goes into solution and is transformed to calcium-malate which, in turn, precipitates out. As the fumarase enzyme sets the malate:fumarate equilibrium of the fermentation mixture to 7:3 molar ratio and calcium-L-malate continuously exits the solution system because of its low solubility, an approx. 100% conversion rate can be reached. The referred solubility data is also true for the magnesium and zinc salts of the said acids. The author has also employed *Lactobacillus delbrueckii* and *E.coli* for the bioconversion. Reaction time was between 16-32 hours and reaction temperature was within the range of 15-45 °C.

Ludwig Degen et al. (DE 2,363,285; 1973) have produced malic acid from fumaric acid employing *Paracolobactrum aerogenoides* belonging to the family of *Enterobacteriaceae* and emphasized the positive effect of adding fumaric acid to the culture medium (enzyme induction effect).

The authors of the patent publication DE 3,434,880 (1986) have converted ammonium-fumarate in their method, present in high concentration. They established that among the mycelium producing fungi examined there were some still working well in an 1.8 M ammonium-fumarate solution and, in contrast to the different bacteria examined, they do not produce amino acids as side products. The selected strains were first pre-cultured and the producing reactor was initiated with 0.5-50 g/l (dried weight) mycelium and the fermentation mixture was stirred and aerated. It was found that the conversion of ammonium fumarate was faster than that of the sodium salt of fumaric acid. The higher solubility of ammonium-fumarate over sodium-fumarate is also advantageous. The described method is suitable for the production of 170-400 g/l natural malic acid. The produced biomass was employed two or three times. The species employed, that were selected by screening, are as follows: *Aspergillus ventii, A. awamorii, Penicillium nalgiovensys, Hypopichia burtonii, Fusarium oxisporum.*

The authors of the Japanese patent publication No. 5,476,804 (1979) prove the advantageous applicability of different yeast strains for producing L(-) malic acid. They most remarkable finding was the fact that the fumarate-malate transformation happens without the production of any side products also when employing different yeast species. They have also used dried biomass immobilized in cellulose acetate membrane as biocatalyst though the half life of the employed continuous production system is not indicated in the publication. The authors employed different yeast strains belonging to the following genera: *Candida, Cloechera, Hansenula, Trigonopsys, Sacharomyces, Endomycopsis, Subaniomyces, Sporomyces, Cryptococcus, Devarrimyces, Nematospora, Pichia, Cruyferomyces, Rhodotorula* and *Thorulopsys.*

Ichiro Chibata and his coworkers have made pioneering research in the field of immobilized biocatalysts. This research has established the development of the continuously producing immobilized cell bioreactors working in industrial alcohol producing plants now in Japan, the productivity of which is 10 to 40-fold higher compared to traditional alcohol producing systems. Japan is also world leader in L(-) malic acid production.

The essence of the first L(-) malic acid production procedure of Chibata et al. was the gentle immobilization of the selected microorganism cells into a polyacrylamide gel [best results were achieved by applying *Brevibacterium ammoniagenes;* European J. Appl. Microbiol. 3: 169 (1976)]. The fixed cell biocatalyst was first treated with a detergent and then filled in a column and a 1 M solution of sodium-fumarate (also comprising a detergent) was then passed through the column. The flow through rate has been optimized. Using the immobilized *Brevibacterium* catalyst, a 430 μmole/g catalyst/h fumarase activity was reached. The cited publication discloses the examination of the effect of different detergents on malic acid production in detail. As an effect of cationic tensides, the succinate dehydrogenase enzyme complex dissociates and, thus, succinate production and, therefore, other side reactions are disabled. Another positive effect of applying detergents is the increase in the permeability of the cell membranes. The pH and temperature optimum of the fixed cell biocatalyst was determined as well as the half life of the continuous production system which was found to be 52.5 days.

The above authors have developed they technology further through years and in 1983 they reported a process [TIBTECH. 1: 1 (1983)] the relative effectiveness of which was 20 times higher than that of the original method. In this new technology they applied a cell friendly immobilizing matrix, kappa carrageenan stabilized by polyethyleneimine. *Brevibacterium flavium* was employed instead of Bre*vibacterium ammoniagenes* and using the immobilized biocatalyst it was possible to develop a bioreactor of 243 days half life on 37 °C. The size of the industrial reactor was 1 m$^3$ and the conversion rate was 70% starting from an 1 M sodium-fumarate solution (pH=7.0) and applying a 450 l/h flow through rate. This procedure is still one of the most advanced industrial L(-) malic acid production process.

E.A. Oliveira et al. report a process for malic acid production using *Sacharomyces cerevisiae* immobilized in polyacrylamide gel particles [Appl. Biochem. Biotechn. 47: 65 (1994)]. The yeast strain applied was genetically manipulated by expressing the fumarase gene and amplifying the copy number of the enzyme in the cells thereby. The productivity of the method was reported to be 60 mmol/g cell/h. This result is much better then the previously reported data and demonstrates the effectiveness of the genetic manipulation approach.

The above data was, however, calculated from reaction rates measured on dilute substrate solutions and is probably not much indicative of the effectiveness obtainable by the method under industrial circumstances where concentrated (1 M or even more concentrated) substrate solutions are applied. Moreover, the authors do not indicate the half life of their biocatalyst and this fact might also indicate that the real (industrially useful) effectiveness of their production system in fact falls behind the values predictable from the published experimental data.

Based upon the above summary of the state of the art it can be assumed that development in the following fields would probably make the industrial scale biotechnological production of L(-) malic acid more effective:

1) Multiplying the copy number of the biocatalyst enzyme within the organism used for production.
2) Optimizing the kinetic parameters of the applied enzyme so as to modify the obtainable L(-) malate:fumarate = 7:3 molar ratio to reach the desirable 9:1 ratio which is highly favorable with respect to industrial downstream processing.
3) Elongating the half life of the applied biocatalyst, i.e. preserving catalytic activity that enhances the overall effectiveness.
4) Enhancing substrate concentration and, thereby, decreasing the total volume of flow through needed for the production of a given amount of L(-) malic acid.

In previous biotechnological methods for the preparation of L(-) malic acid, the level of the fumarase enzyme in the applied microorganisms was increased by adding fumaric acid to the medium as the only carbon source, inducing the expression of the enzyme thereby.

There is another known approach for increasing the copy number of the fumarase enzyme in the applied cells, namely the addition of competitive inhibitors of it to the nutrient medium which also induces the expression of the enzyme. The response to the biochemical enzyme induction, however, is strictly regulated in the cells so it is not possible to enhance the copy number of the biocatalyst by more than an order of magnitude using this approach. A higher increase in the enzyme copy number is only obtainable by the means of genetic engineering.

In 1995, we have elaborated a new biotechnological method for the production of L(-) malic acid starting from fumaric acid using recombinant bacteria overproducing the fumarase C enzyme of *E.coli* [Hungarian patent application No. P9402689 (September 19, 1994), laid open for public inspection: June 28, 1996]. This method has two major advantages over the previously known processes.

First, the amplified biocatalyst applied was the fumarase C enzyme of *E.coli* the kinetic parameters of which resemble that of the eucariotic mitochondrial fumarase enzymes and the chemical equilibrium is, thus, set to the industrially desirable 9:1 molar ratio in favor of L(-) malic acid.

As it will be discussed later, this equilibrium value, however, cannot be reached in industrially useful production systems as conversion rate of optimal flow through rate bioreactors working with high substrate concentration can not exceed 80%.

Secondly, amplification of the copy number of the applied enzyme was reached by employing constitutive expression vectors (pCKR101/fumC, pRK290/FumC, described in: Hungarian pat. appl. No. P9402689, see above). This is a big advantage as, thus, recombinant cells comprising the appropriate expression vector express the enzyme throughout their whole life cycle.

It is known that genetically manipulated microorganisms usually produce the desired protein the most intensively in the so called early log phase, since at this phase the nutrient medium used for replication is rich in substances (e.g. amino acids) needed for biosynthetic reactions and general regulatory mechanisms of the cellular metabolic system favor the synthetic pathways.

At this phase, the number of cells in the medium is relatively low (about $10^5$-$10^7$ cells/ml) making the production of biocatalysts of proteinaceous nature uneconomical.

The concentration of the biomass reaches the concentration of $10^{10}$ cells/ml at the end of a good fermentation. This high cell concentration means at least a 100,000 fold increase in the copy number of the biocatalyst compared to that in the early log phase - taken the whole biomass into account (as the produced biomass can also be immobilized for L(-) malic acid production in intact form) - and overproduction of the enzyme within the cells still adds to this amplification.

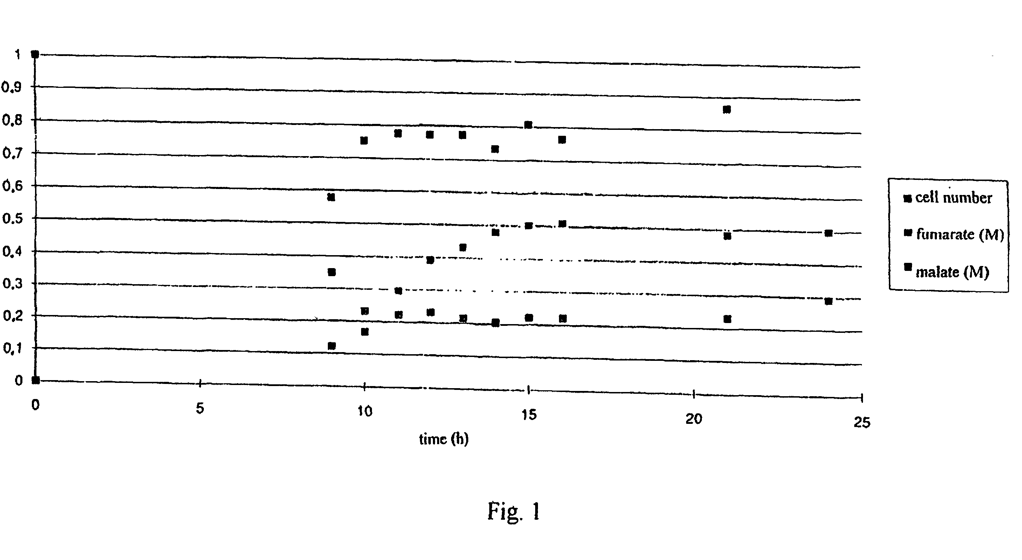

Continuous expression of the enzyme from the constitutive expression plasmids, thus, enables the production of maximum amount of biomass during the scale up procedures as there is no need for harvesting the cells early from the fermentor. It becomes also possible to continuously produce the cells for the preparation of the fixed cell biocatalyst by continuous fermentation as it can be seen from our recent results (see Fig. 1). On Fig. 1, it is clearly demonstrated that within the examined time interval, cells do not lose - even in the late log phase - their enzymatic activity or the plasmid expressing the enzyme.

The third possibility of enhancing the effectiveness of L(-) malic acid production is significantly reducing the volume requirement of the biocatalysis while retaining unchanged mass flow, i.e. developing a continuously producing L(-) malic acid production process.

This can be achieved through immobilizing the enzyme producing cells. Based on published experimental data an on the above arguments, one can assume that by immobilization of the producer cells, the effectiveness of the process can be increased 10-40 fold compared to a free cell type batch fermentation. In industrial alcohol producing systems, for example, the best productivity of traditional batch fermentation is reported to be 2.2 kg/m$^3$h ethanol while this value can go even up to 50.4 kg/m$^3$h when applying continuously producing fixed cell bioreactors.

Among the known immobilization methodologies the so called "inclusion" techniques seem to be the most economic as these methods enable the immobilization of the highest amount of biomass per volume. The common feature of the different inclusion methods is the use of gels having large inner pores for immobilizing living cells.

The industrial requirements for gels used for the immobilization of biocatalysts are as follows:

- high operational stability and inertness (the gel must not chemically react with any substance participating in the biotechnological process);
- sufficient mechanical rigidity;
- suitable macro- and microporosity;
- cell friendly immobilization technique.

And last but not least, the costs of immobilization must be in harmony with the price of the desired product achievable on the market.

None of the known gel structures meet all the above requirements at the same time, therefore, we decided to elaborate a new immobilization method suitable for developing our L(-) malic acid production technology further.

Inclusion methods employing so called "soft" gels can be separated into two groups. Techniques belonging to the first group employ polymeric substances for preparing the gel matrix that make the recovery of the immobilized material from the gel structure possible.

These methods include the so called "ionotropic" inclusion methods in which different metal ions are used fix the gel structure made of different negatively charged polymers (e.g. alginates and pectins). Methods are also known where polycationic gel structures are stabilized by the use of organic acids. The common advantage of these techniques is the high preservation of the biological activity as these methods are very gentle one step procedures. A common disadvantage of the said methods is, however, the fact that the long term operational stability of the prepared gel structures can only be preserved by maintaining the required cation concentration throughout the whole fermentation process. This problem significantly reduces the applicability of the biocatalysts immobilized by the ionotropic methods.

The second group of immobilization techniques consists of methods in which the original components can not be recovered without alteration from the immobilizing matrix. Such methods employ biocatalysts immobilized by different polymerization techniques in e.g. polyacrylamide gels or polyurethane foams.

The common disadvantage of these methods is the significant loss of biological activity during the immobilization process and, moreover, the enclosed biological activity decreases faster than in other immobilization methods due to the natural aging processes of said polymeric gels. Enzymes immobilized in polyacrylamide gels, for example, usually lose 70-95% of their biological activity during the inclusion and the half life of the enclosed activity is generally about 30-90 days.

A person skilled in the art can, therefore, appreciate that preparing a novel gel structure meeting all the above requirements at the same time, without serious disadvantages, is not an easy task.

Finally, WO 93/24112 discloses microcapsules for the use in encapsulation of living cells, wherein said microcapsules are made of a mixture of two types of alginate, one with higher mannuric acid content(M alginate) and one with higher guluronic acid content (G alginate). The capsules are formed in the presence of $Ca^{2+}$ and coated with poly-L-lysine.

It is, thus, an object of the present invention to provide a novel highly efficient biotechnological method for the preparation of L(-) malic acid from fumaric acid based on the employment of a novel immobilized biocatalyst giving industrial scale productivity even using laboratory equipment for the production.

Using such an improved technology makes possible to spare a large portion of the usual fermentation scale up costs that in most cases amount to 75-90% of the total development costs of new biotechnological processes.

The environmental protection effect of such an intensive biotechnological method compared to the present chemical production processes is also to be noted as it comprises less operational steps and produces optically clear malic acid from fumaric acid which can be considered as an industrial byproduct.

The object of the invention was achieved by elaborating a novel highly effective immobilization technique making possible the operation of the fixed cell biocatalysts prepared by the method of the invention by significantly longer half life. The novel reactor packing prepared by the method of the invention is also operable as killed cell type biocatalyst (i.e. enzyme biocatalyst) as a result of the special coating formed on the special composite gel particles of the invention - in which the cells are immobilized - that prevents the leakage of the enzyme from the reactor packing.

The invention is based on the finding that molecular composite gel particles of extremely advantageous characteristics (improved elasticity, mechanical and osmotic stability and ionic resistance) suitable for application as a reactor packing can be prepared of alginate combined with a polyanion of similar structure to agarose but also comprising acidic side groups (e.g. GELRITE™, Kelco, USA) with the use of a suitable complexing agent and bivalent metal ions. On the surface of the composite gel particles of the invention it is very simple to form a coating membrane of a predetermined pore size by the use of polycations (e.g. chitosan).

The basic idea leading to the development of the new immobilization technique of the invention comes from the similarities and differences between the molecular structures of agarose and GELRITE™ (which is a biopolymer commercialized as an agarose substitute).

Agarose is a linear homopolymer built from glucose units joint to one another by $\alpha$-1-4 bonds. Agarose can be dissolved by heating and it has a definite gelling temperature. The gelling temperature of the different agarose types depends on the frequency of occurrence of sulphonated glucose units in the polymer. Sulphonated glucose units bound together by the contaminating polyvalent cations present in trace amount in the system serve as crystal formation focus. The lose helical type molecular structure - the so called "disordered region" - of the forming gel starts to build up from the above mentioned crystalling foci.

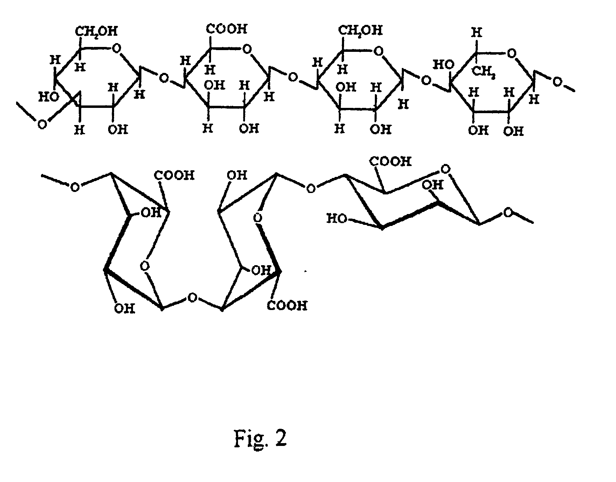

The molecular structure of GELRITE™ is closely related to that of agarose but it comprises more groups of acidic character. Namely, GELRITE™ consists of a repetitive sequence of the - (D-glucose-guluronic acid-D-glucose-L-rhamnose)- structural unit (see Fig. 2). GELRITE™ has a definite gelling temperature like agarose. The mechanical strength of 0.8-1.5% GELRITE™ gels corresponds to that of 2-4% agarose gels. GELRITE™ gels are neither sensitive to the ionic strength of the medium nor to the ratio of mono- and bivalent cations in contrast with the ionotropic gels. GELRITE™ gels are more rigid than agarose gels and when heating above their gelling temperature under physiological conditions, they do not dissolve but start to shrink while losing water. This characteristic of GELRITE™ gels resemble to that of alginate gels and limits their practical applicability, too. So far GELRITE™ gels were only used for solidifying nutrient media at temperatures where agar and agarose gels are not stable (in the range of 40-85 °C).

It was surprisingly found, however, that in the presence of complexing agents liquid solutions can be prepared from GELRITE™ at room temperature and even below, just like from alginate. This gel forming material was, thus, also made suitable for cell immobilization purposes. GELRITE™ is suitable for this purpose in the 0.05-4.01% (dried material) concentration range but only if a complexing agent (e.g. EDTA) is present in the solution in excess.

As a consequence of the above finding, GELRITE™ gels for immobilization purposes can now be prepared by simple dropping or fiber pulling methods like alginate gels. Namely, the biomass is suspended in the matrix forming solution and the suspension is added dropwise to a solution of a suitable cation (under physiological circumstances it is advisable to use a 0.005-0.2 M solution of $CaCl_2$). Gelling occurs as a consequence of the diffusion of the metal ions towards the inner space of the drops as in the case of alginate gels. The rigidity of GELRITE™ gels can be decreased by the use of complexing agents through which it is also possible to cease the gelling point.

The optimization of the characteristics of the new immobilization matrix was reached by mixing GELRITE™ solutions with alginate solutions of different concentrations (also comprising complexing agent). After dropping the mixture in a gelling solution comprising $Ca^{2+}$ ions, we could create gel particles of new molecular composite structure in a wide concentration range (0.05-4.1%, dried weight).

The molecular composite gels of the invention are osmotically stable, they are not sensitive to the ionic strength or distribution of the medium and, at the same time, they are elastic, and the mechanical strength of them exceeds the strength of gels of similar concentration prepared solely from one or another of the composite gel forming materials. Their operational stability is excellent as after more than 365 days of continuous production, the mechanical strength of the composite gel particles is not reduced significantly.

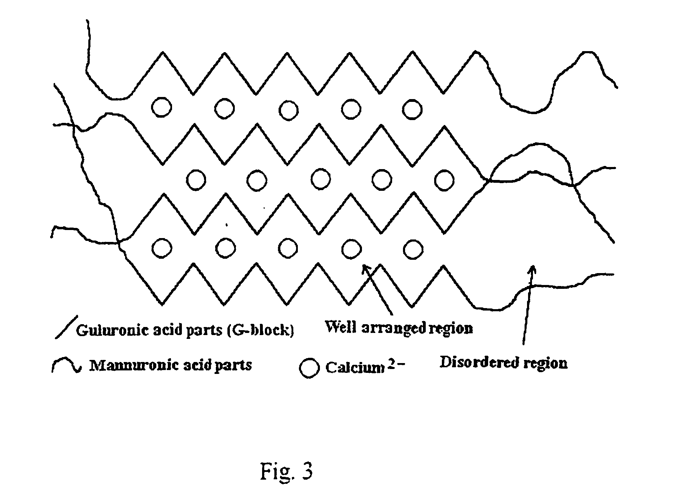

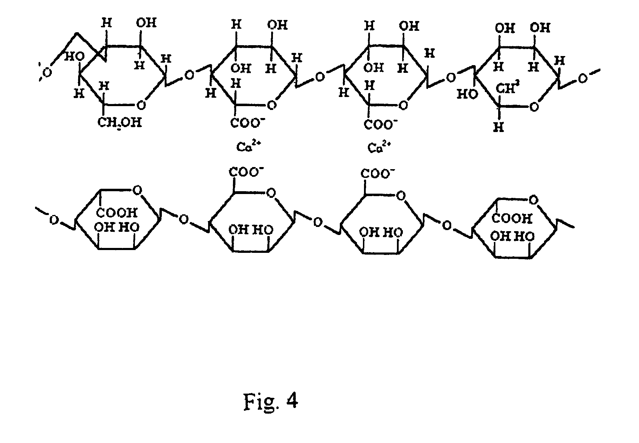

Though not wishing to be bound by any theory, the probable molecular explanation of these advantageous characteristics of the gels of the invention lies in the fact that GELRITE™ molecules can build into the ordered regions of alginate gels (see Fig. 3) without significantly disturbing the regions formed of the guluronic acid homopolymer regions of the alginate molecule bound together by intermolecular salt bridges formed by calcium ions (see Fig. 4).

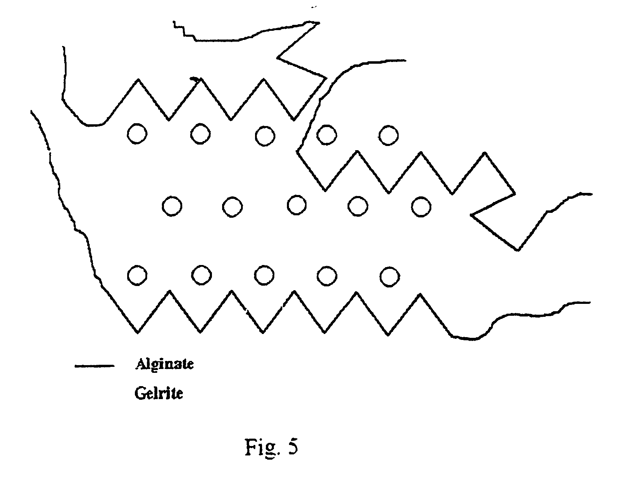

As in GELRITE™ molecules every fourth unit is a guluronic acid on an average, they have good chances for being built in the linear regions of the ordered domain thereby loosening said regions. GELRITE™ molecules can be built into more than one ordered region of the gel structure at the same time, situated at different spatial points (see Fig. 5). Moreover, the chance that the alginate or GELRITE™ molecules form ordered regions among themselves is lowered, as the presence of the partner molecule in a given spatial region decreases the relative concentration of the other type of molecules there, and the rigidity of the structure is enhanced thereby.

It is also probable that the average length of the "egg box" structural elements characteristic of alginate gels is decreased and their average frequency of occurrence increases (the guluronic acid homopolymer blocks in alginate gels usually consist of 10-12 units). This phenomenon may explain the enhancement of the elasticity of the composite gel compared to pure alginate gels.

As guluronic acid units of the GELRITE™ molecule can not form intermolecular joints with the so called "M box" region of the alginate gel, the average size and frequency of occurrence of the disordered regions of the structure remains probably unchanged and, therefore, the inner pore size of the novel composite gels remains unaltered compared to pure alginate gels.

As inner porosity of soft gels - and also the diffusion rate of substrates and products - is ultimately defined by the structure of the disordered regions, it is a very important fact that the structure of said regions remains unaltered in the composite gels.

As shown in our experiments (data not shown), a protein of algae origin having a maximal molecular mass of 800,000 D is still capable of diffusion within alginate and alginate containing composite gels. However, the effective catalyst comprised in the biocatalyst particles of a fixed cell L(-) malic acid producing systems is the fumarase enzyme having an average subunit size of 50,000 D.

It will be easily appreciated by the person skilled in the art that, though, usually living cells are immobilized within the biomatrix, during the operation of the system (and also during the immobilization process itself) cells get slowly disrupted or lised and the biocatalyst enzyme can diffuse into the medium. The half life of the production system is, thus, eventually determined by the effectiveness of the enclosure of the enzyme within the gel particles. The fumarase C enzyme having a molecular mass of 50,000 can, for example, easily leave the usual alginate based immobilizing gels.

To solve this problem, we have formed an asymmetric membrane structure *in situ* on the surface of the novel alginate-GELRITE™ composite gel particles by the use of high molecular weight chitosan. The average pore size of the coating membrane was so small that it was impermeable for bovine serum albumin having a molecular mass of 50,000 D.

Surface modification of polyanionic gels by polycationic substances is a method known *per se* but the process had to be carefully optimized so as to form a coating membrane of the desired average pore size.

The present invention, therefore, provides an immobilized biocatalyst comprising living cells and/or parts thereof wherein

    i) said cells and/or parts thereof are comprised in gel particles of molecular composite structure built from at least two different polyanionic substance bound together by polyvalent cations, advantageously by bivalent metal ions;

i) said cells and/or parts thereof are comprised in ionic strength resistant, osmotically and mechanically stable gel particles of molecular composite structure built from at least two different polyanionic substances bound together by polyvalent cations, advantageously by bivalent metal ions; one of said polyanionic substances being an ionotropic gel forming material and another of said polyanionic substances being a gel forming material having a definite gelling temperature;

ii) optionally, a membrane of a predetermined average pore size is formed around said composite gel particles by the use of a polycationic substance; and

iii) optionally, the cells comprised in said gel particles are killed by a treatment with a detergent and/or by sonication after being immobilized in the gel particles.

The immobilized biocatalyst according to invention. preferably comprises gel particles that are built from alginate and a polyanionic substance of agarose related structure comprising anionic side groups, too - e.g. the artificial agar named GELRITE™ (Kelco, USA) - and, optionally, the gel particles are coated by a membrane formed by using chitosan and, optionally, the immobilized cells are killed by a treatment with Na-cholate and/or by sonication.

The gel particles of the invention are advantageously coated by a membrane not permeable for globular proteins of 50,000 D molecular mass.

The immobilized biocatalyst according to the invention preferably comprises gel particles comprising recombinant *E.coli* cells - and/or parts thereof - transformed with a vector constitutively expressing fumarase C enzyme, advantageously with plasmid pCKR101/FumC or derivatives thereof. The immobilized biocatalyst of the invention advantageously comprises 5-20% biomass.

The invention also provides a process for producing L(-) malic acid from fumaric acid by applying the immobilized biocatalyst of the invention for transforming fumaric acid to L(-) malic acid. The process of the invention is advantageously practiced in continuous production manner at 35 °C, starting from a solution comprising at least 1 mol/l fumaric acid.

The invention is further illustrated by the attached figures the short description of which is as follows:

The graph on Fig. 1 shows the time dependence of the specific fumarase activity of recombinant *E.coli* DH5-$\alpha$ cells transformed with the plasmid pCKR101/FumC during a 25 hour fermentation.

Fig. 2 shows the molecular structure of the artificial agar named GELRITE™ (Kelco, USA).

Fig. 3 shows the so called "egg box" model of the structure of alginate gels.

On Fig. 4 we show the putative structure of the alginate-GELRITE™ composite gels.

Fig. 5 shows the putative "egg box" model of the structure of alginate-GELRITE™ molecular composite gels.

Fig. 6a and 6b demonstrate the results of our experiments for determining the protein permeability of the coating membrane of 2% alginate-GELRITE™ gel particles coated by applying chitosan solutions of different concentrations. On the vertical axis, the extinction of BSA remained in solution after removing the gel particles is shown.

On Fig. 7 we show the putative structures of alginate-GELRITE™ gel particles coated by chitosan solutions of different concentrations.

Fig. 8 shows the operational stability of bioreactors packed with intact cell and lised cell comprising biocatalysts during a 140 days interval.

Fig. 9 is a graph showing the results of flow rate optimization experiments on a bioreactor packed with a biocatalyst comprising recombinant *E.coli* cells transformed with plasmid pCKR101/FumC.

Fig. 10 is a graph showing the results of flow rate optimization experiments on a bioreactor packed with a biocatalyst comprising halophyl *Micrococcus roseus* bacteria.

Fig. 11 is a graph showing the comparison of the specific activity of bioreactors packed with biocatalysts comprising recombinant *E.coli* cells transformed with plasmid pCKR101/FumC or halophyl *Micrococcus roseus* bacteria.

Fig. 12 is a graph showing the results of an experiment for determining the half life of the bioreactor packed with a biocatalyst comprising recombinant *E.coli* cells transformed with plasmid pCKR101/FumC operated at constant flow rate.

Fig. 13 is a graph showing the results of optimizing the biomass content of the bioreactor packed with a biocatalyst comprising recombinant *E.coli* cells transformed with plasmid pCKR101/FumC.

Fig. 14 is a graph showing the L(-) malic acid productivity of the bioreactor packed with a biocatalyst comprising recombinant *E.coli* cells transformed with plasmid pCKR101/FumC in the 0-60 °C temperature range.

Fig. 15 is a graph showing the results of an experiment for determining the temperature optimum of the bioreactor packed with a biocatalyst comprising recombinant *E.coli* cells transformed with plasmid pCKR101/FumC.

The subject of the invention will be further illustrated by the following experimental examples, however, the scope of the invention will by no means be limited to the specific advantageous embodiments described in the examples.

Example 1

Preparing composite gel particles coated by a membrane of optimal pore size

First, we have prepared a 2% (total dried weight) alginate-GELRITE™ polymeric matrix as follows.

A Na-alginate (Protanal HF) solution is prepared in 100 ml quantity by dissolving the alginate in a 100 mg/ml solution of EDTA (pH=7). Similarly, 2% GELRITE™ is suspended in 100 ml 100 mg/ml solution of EDTA. The solutions are then autoclaved for 20 min.

The hot sterile solutions are then mixed and the mixture is left to cool to room temperature. Through a sylanized capillary, 20 ml of the mixture is then dropped into 100 ml of a 50 mM $CaCl_2$ solution while gently stirring the solution. In the applied $CaCl_2$ solutions, chitosan was solved previously in different concentrations (0.0001-0.5%). After drop forming, the $CaCl_2$ solution is still slowly stirred for 30 minutes and the solution comprising the gel particles is kept overnight in a refrigerator. Gel particles also comprising biomass are produced as follows. The cell culture being in the desired phase is centrifuged by 6000 rpm at 4 °C and the weight of the biomass is determined. An amount of physiological salt solution (0.85% NaCl) corresponding to 5% of the total wet weight of the biomass is then added and the cells are resuspended (by gentle mixing).

The above concentrated cell suspension is then mixed in the desired amount (5-20% v/v) with the above gel forming solution and then the cells are immobilized by forming gel particles as above.

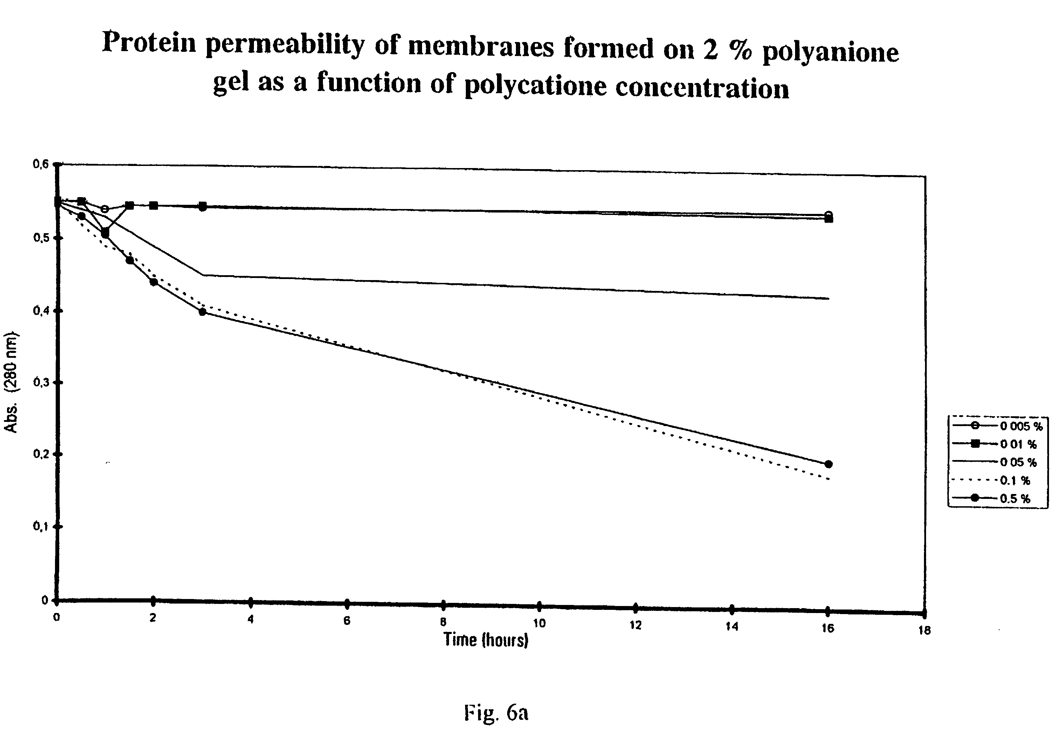

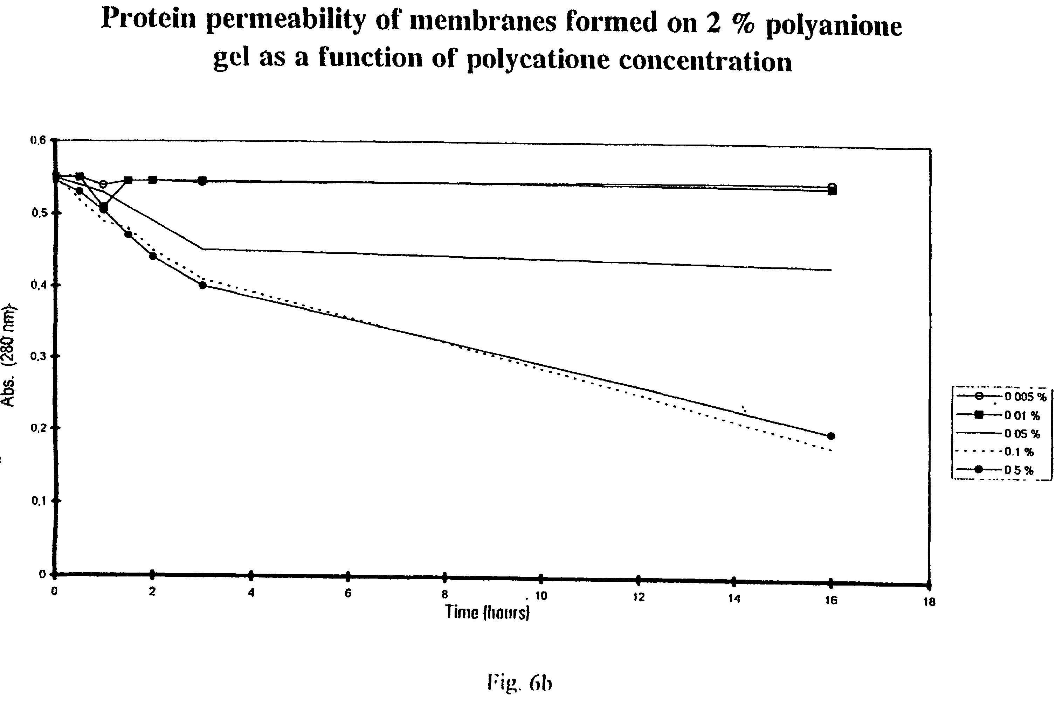

The permeability of the formed coating membrane can be checked by 5g of the coated particles to 10 ml of 1 mg/ml BSA solution, the suspension is gently mixed at room temperature and the change of the extinction of the outer solution with time is measured (see Fig. 6).

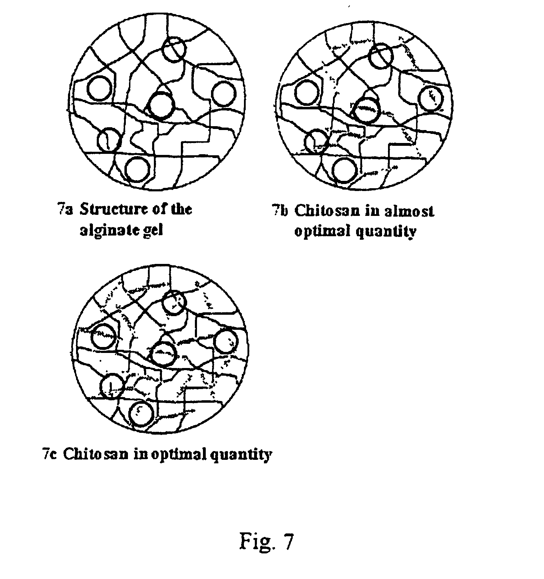

As it can be seen on Fig. 6, the permeability of the formed coating membrane highly depends on the concentration of the applied cationic polymer and only treatment with 0.005% chitosan results in a coating membrane practically impermeable for BSA. Within the examined wide concentration range, only treatment with chitosan in optimal concentration resulted in BSA impermeable coating membrane. The probable explanation of this phenomenon is modeled on Fig. 7.

According to the model shown, the positively charged chitosan is bound to the disordered regions of the gel structure as available free carboxylic groups are only there, and the polycation, thus, pulls these regions closer together. When applying the polycation in suboptimal concentration, there remain pores of original size on the particles through which proteins are capable of diffusion (see Fig 7b) while the polycation is used up from the solidifying $CaCl_2$ solution. In optimal concentration, chitosan coats all the original pores on the surface of the particles (Fig. 7c). When applied in supraoptimal concentration, the coating membrane deforms the original structure so much that it tears and large pores are formed again through which BSA can again penetrate. (BSA molecules are symbolized on the figure with circles).

Example 2

Preparation of an immobilized biocatalyst comprising killed *E.coli* cells

It is advantageous from industrial (mainly from environmental protection) viewpoint if an immobilized biocatalyst does not comprise living cells. Technologically it is advantageous to immobilize the intact cells, packing the biocatalyst into the reactor and then killing the cells *in situ.*

The most obvious choice for killing the immobilized cells is the treatment with detergents. This statement is supported by the facts that said treatment on the one hand loosens the structure of the membranes of the immobilized cells and, on the other hand, irreversibly disrupts the succinate dehydrogenase enzyme complex in the cytoplasm which inhibits non desired side reactions (see Chibata et al., supra).

Below we describe the experiments done for optimizing the quantity of the detergent to be used for killing the immobilized bacteria.

First, an *E.coli* strain transformed with the constitutive expression plasmid pCKR101/FumC was cultured. After 16 hours of culturing, cells were spin down at 4 °C and the biomass was divided into two aliquots. One aliquot was immobilized according to the method set forth in Example 1, the immobilized biocatalyst comprised 20% biomass. The other aliquot was resuspended in a 10% solution of Na-cholate and the suspension was then sonicated on ice for 3 minutes applying 3 seconds impulses with 3 seconds pauses between pulses. After sonication, the biomass was immediately immobilized. The two differently prepared immobilized biocatalyst were packed into bioreactors of 11 ml effective volume. Both reactors were washed with two column volume of 1 M Na-fumarate solution comprising 5% Na-cholate with 2 hours residence time before operation. The substrate was the replaced with an 1 M Na-fumarate solution (pH=7.0) and the reactors were operated through 100 days with a residence time of 2.37 h at room temperature.

The other part of the immobilized biocatalyst was divided into 5 g aliquots and given into 1 M Na-fumarate solutions also comprising Na-cholate in different concentrations and the amount of L(-) malic acid produced was determined.

Microbial analysis was also performed on the applied biocatalyst. Sampling was done in sterile conditions and 0.1 ml of the reaction mixtures were plated on agar plates.

In the case of bioreactors, samples were taken from the effluent in every 20 minutes during the course of the detergent treatment and were plated as above. As the composite gel particles used were not soluble, the number of living cells in the particles was determined by rubbing two particles (approx. 0.1 ml) in every sampling under sterile conditions and the disrupted particles were plated.

Data of Table I below show that as a consequence of treating the reactors together with Na-cholate and 1 M Na-fumarate they become sterile in 48 h and this ceases the potential hazard of the leakage of viable genetically manipulated *E.coli* cells into the environment.

Table I

| Time (h) | No. of living cells in the gel particle [log(cell no./ml)] |
|----------|-----------------------------------------------------------|
| 1 | 8.48 |
| 3 | 9 |
| 5 | 8 |
| 7 | 7.49 |
| 25 | 0 |

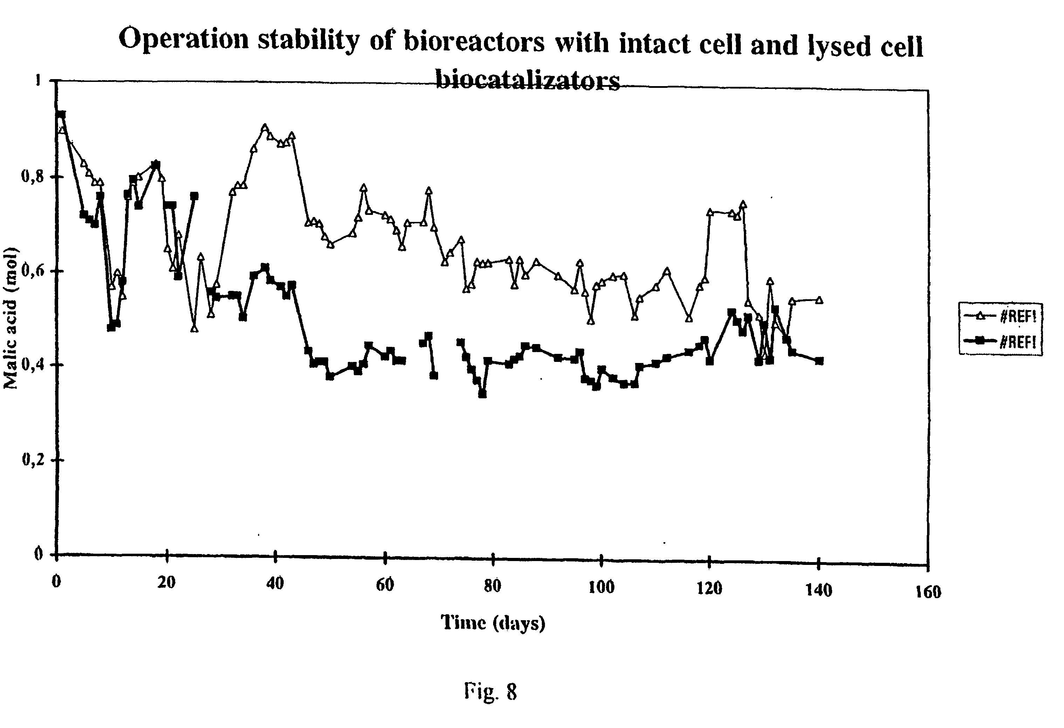

The above experiment also revealed another important fact, namely, immobilizing intact cells is much more effective in long term then immobilizing cell lisates. As it can be clearly seen on Fig. 8, immobilized biocatalyst comprising cells that were immobilized in intact state loses its bioactivity much slower than its counterpart comprising immobilized cell lisate, though both reactors has the same starting activity.

It can be seen from the data of Table II below that the treatment with detergent solutions of different concentrations is practically ineffective on the biocatalyst comprising immobilized crude cell lisate. In the case of the biocatalyst comprising cells immobilized in intact state, however, a significant increase in the reaction rate can be seen as an effect of Na-cholate applied in a concentration as low as 0.5%. Increasing the concentration of the detergent, however, has no further effect either on the reaction rate or on the maximum amount of L(-) malic acid produced measurable in the equilibrium state system.

EP 0 931 142 B1

Table II.a

Intact cell systems

Sampling time (min)

| Na-cholate conc. (%) | 0 | 20 | 40 | 60 | 80 | 100 | 120 | 140 | 160 | 180 |
|---|---|---|---|---|---|---|---|---|---|---|
| Control | 0 | 0.072 | 0.263 | 0.349 | 0.52 | 0.508 | 0.608 | 0.546 | 0.6 | 0.609 |
| 0.5% | 0 | 0.212 | 0.393 | 0.495 | 0.55 | 0.563 | | 0.619 | 0.561 | 0.575 |
| 2% | 0 | 0.274 | 0.414 | 0.578 | 0.604 | 0.59 | | 0.598 | 0.624 | 0.575 |
| 5% | 0 | 0.281 | 0.418 | 0.568 | 0.56 | 0.577 | 0.554 | 0.558 | 0.585 | 0.563 |
| 10% | 0 | 0.272 | 0.358 | 0.55 | 0.581 | 0.559 | 0.514 | 0.524 | 0.565 | 0.633 |

Table II.b

| Lised cell systems Sampling time (min) | | | | | | |
|---|---|---|---|---|---|---|
| Na-cholate conc. (%) | 0 | 20 | 40 | 60 | 80 | 100 |
| Control | 0 | 0.348 | 0.561 | 0.695 | | 0.692 |
| 0.5% | 0 | 0.388 | 0.585 | 0.679 | 0.73 | 0.697 |
| 2% | 0 | 0.398 | 0.561 | 0.65 | 0.717 | 0.702 |
| 5% | 0 | 0.376 | 0.579 | 0.637 | 0.695 | 0.699 |
| 10% | 0 | 0.4 | 0.563 | 0.618 | 0.646 | 0.679 |

Results shown in this example demonstrate that one treatment of packed bioreactors with a substrate solution comprising maximum 2% detergent is necessary and enough. This treatment also prevents the leakage of viable recombinant cells into the environment.

Example 3

Optimization of flow through rate in a bioreactor packed with an immobilized biocatalyst comprising

With the purpose of determining the optimal flow rate and the half life of the immobilized biocatalyst of the invention two L(-) malic acid producer reactors of similar effective volumes (11 ml) were started to operate at room temperature, the substrate concentration was 1 M. One of the reactors were operated at constant flow rate. The flow rate of the other reactor was changed once a week on average. The starting flow rate was set to define a 3 hours residence time (that is the usual residence time at biotechnological operations) and it was increased to reach a residence time of 0.18 h which is shorter than the diffusion time (20 min) measured on the gel particles having a mean diameter of 2.4 mm.

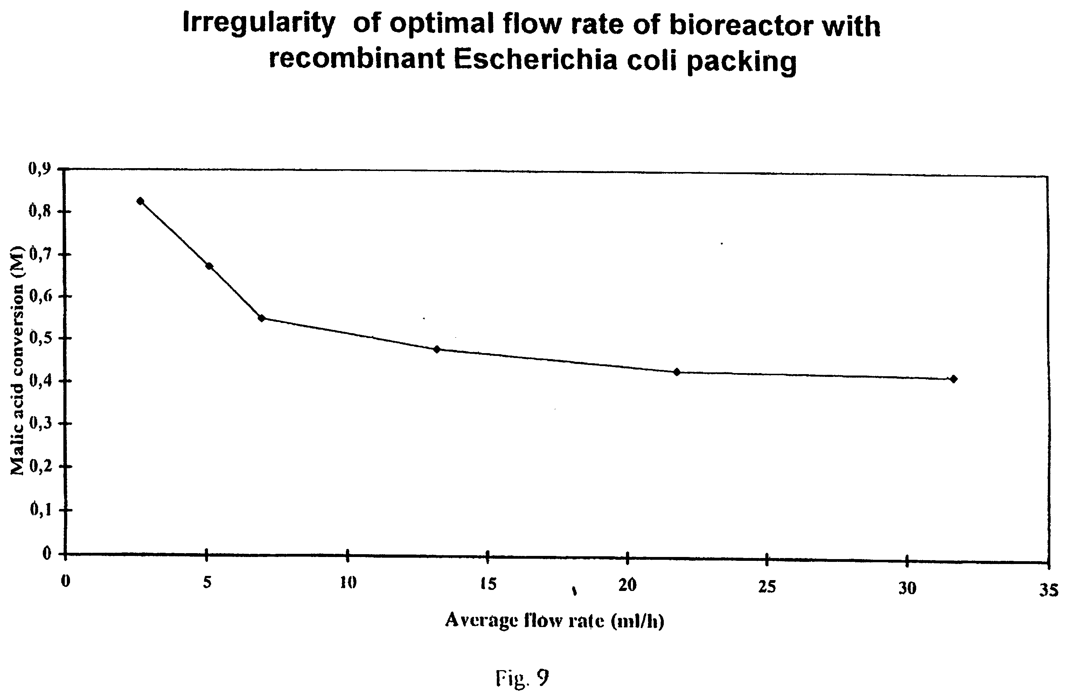

The average of the conversion rates (which in this case equals with average of the molar amounts of L(-) malic acid determined in the effluent) was calculated. The mean conversion rate values were plotted against the actual flow rate values (Fig. 9).

As it can be seen on Fig. 9, the curve has no bending point hinting to the fact that the conversion rate above a given flow rate would be linearly reduced with the enhancement of the flow rate. The optimal flow rate, thus, can not be determined unambiguously. Even at a flow rate as high as 30 ml/h, the conversion rate of the system is still about 50%.

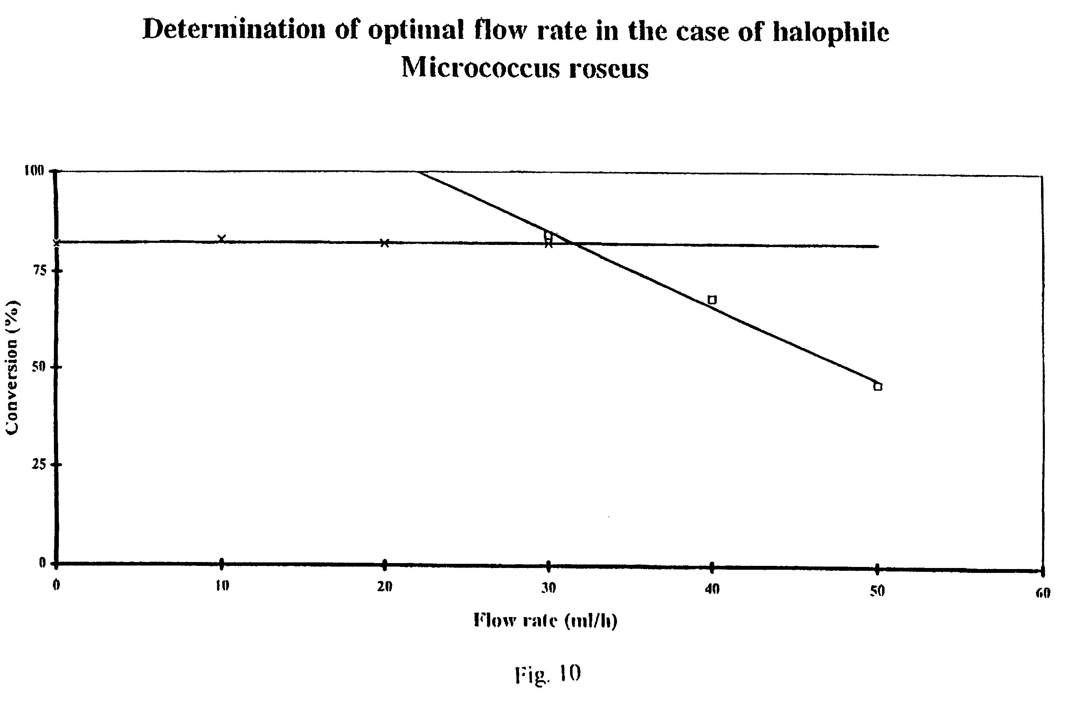

This finding is in sharp contrast with the experimental data known from the biotechnological practice when using genetically unmodified bacteria. We have also made similar experiments using a genetically unmodified halophyl *Micrococcus roseus* isolate. This experimental system produced a curve with a definite bending point and the optimal flow rate could be unambiguously calculated. This value was determined to be 33 ml/h for a bioreactor of 100 ml effective volume (Fig. 10).

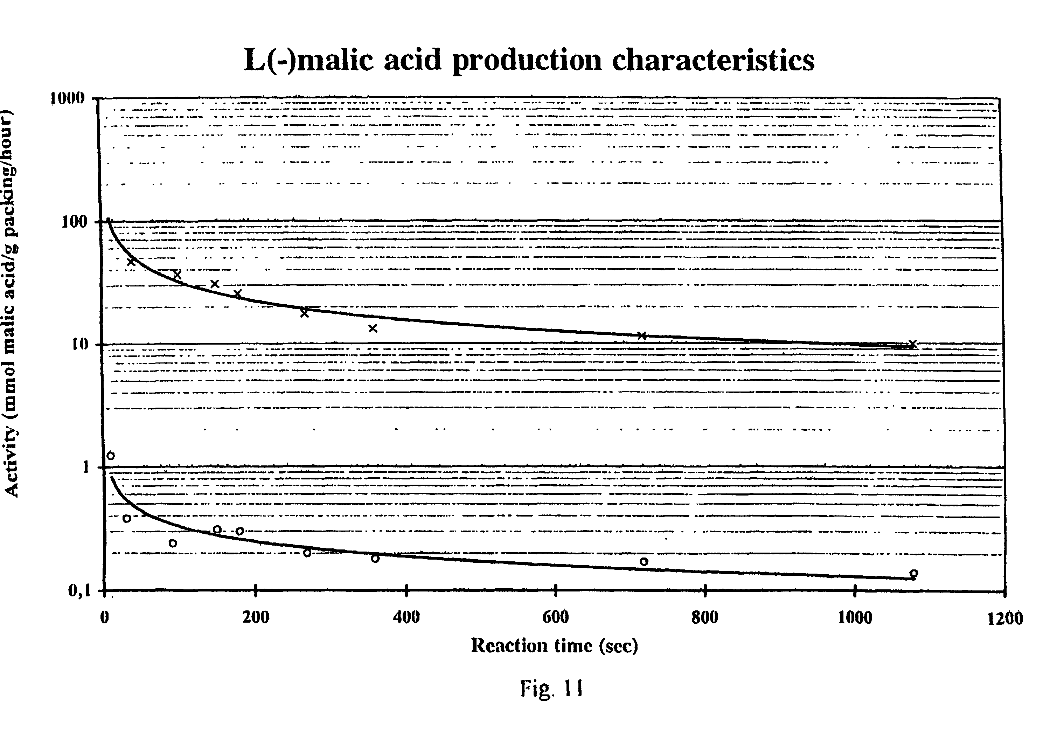

So as to figure out the explanation of the kinetic anomaly found in the recombinant *E.coli* system, we have plotted the specific activity values - belonging to the given range flow rates - of both systems against the residence time values (Fig. 11).

As it can be seen on the graph shown on Fig. 11, the specific activity of the *Micrococcus* based system decreases hyperbolically with the residence time. The specific activity of the recombinant system, however, decreases linearly with the residence time. This fact unambiguously indicates that fumarase enzyme molecules in the recombinant system do not work at substrate saturation state even at extremely high flow rates, probably because of the very high copy number of the biocatalyst immobilized. It is true, at the same time, that conversion rate in the recombinant system does not reach the theoretical maximum (a value above 90%) even at very low flow rates because, during the long residence time, the back transformation of the product is enhanced, too.

It was, thus, established that the immobilized biocatalyst of the invention comprising recombinant cells can not work at maximum capacity in the flow rate range examined, i.e., it is "too good".

Example 4

Optimizing the biomass content and determining the half life of the bioreactor packed with immobilized biocatalyst comprising recombinant *E.coli* cells

In view of the results shown in Example 3, it was necessary to optimize the biomass content of the recombinant system.

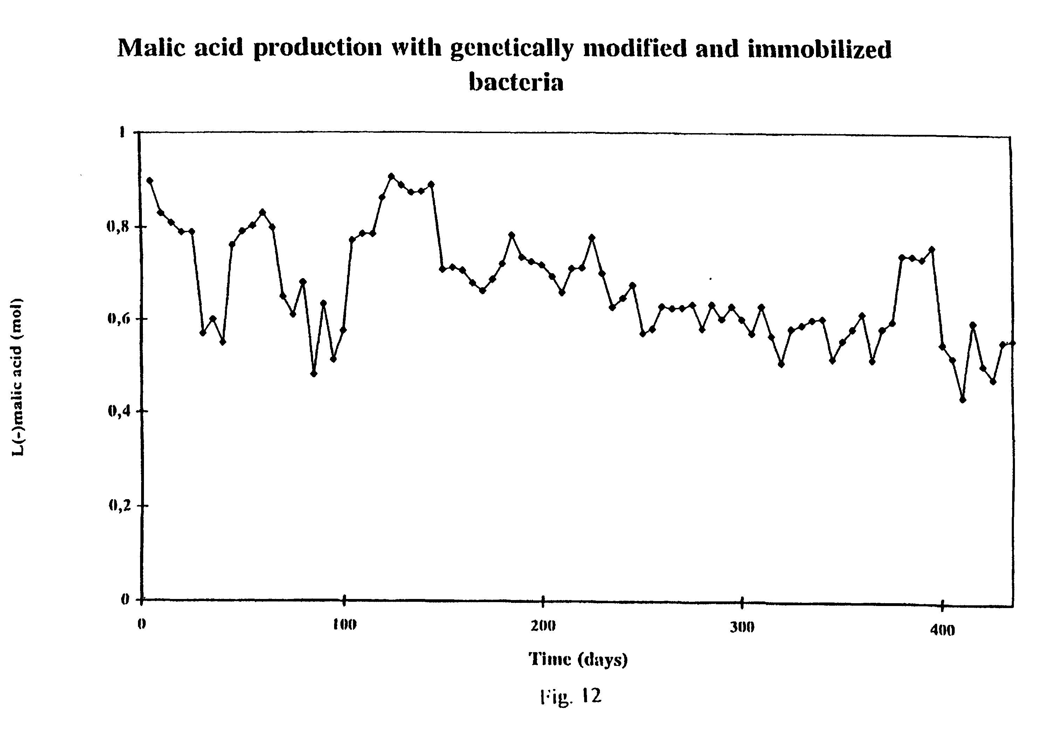

The logarithm of the conversion rate of the constant flow rate bioreactor was plotted against the experimental time and the half life of the recombinant system was, thus, determined (Fig. 12). The determined value of 210 days (at room temperature) is significantly lower than the half life of the immobilized biocatalyst comprising non recombinant living cells (370 days). This "loss", however, is well compensated by the 40 fold specific activity of the recombinant system.

After 50 days of operation, the two bioreactors of Example 3 were further operated applying an 1.5 M ammonium-fumarate solution as substrate (see Table III below). As it can bee seen from the data given in Table III, the average conversion rate calculated from the values measured between the 51st and 62nd days of operation was 0.678. This indicates that the enclosed amount of enzyme does not operate at substrate saturation state even at a substrate concentration of 1.5 M. This result indicated the further internal reserves of the system.

Table III

| Effect of ammonium-fumarate substrate on the bioconversion | |
|---|---|
| Sampling (days) | Malate produced |
| 0 | 0.68 |
| 1 | 1.1 |
| 3 | 1.05 |
| 4 | 0.98 |
| 10 | 0.99 |
| 12 | 1.00 |
| 28 | 0.76 |
| 48 | 0.75 |
| 54 | 0.7425 |
| 61 | 0.6141 |
| 75 | 0.7471 |

In new independent batch type experiments, we have re-optimized the amount of biomass to be enclosed in the composite gel particles of the invention. Immobilized biocatalysts were prepared as in Example 1, comprising 1, 5, 10, 15 and 20% v/v recombinant *E.coli* cells, respectively. Five grams of the catalysts were tested at room temperature in 50 ml 1 M Na-fumarate solution comprising 0.5% Na-cholate.

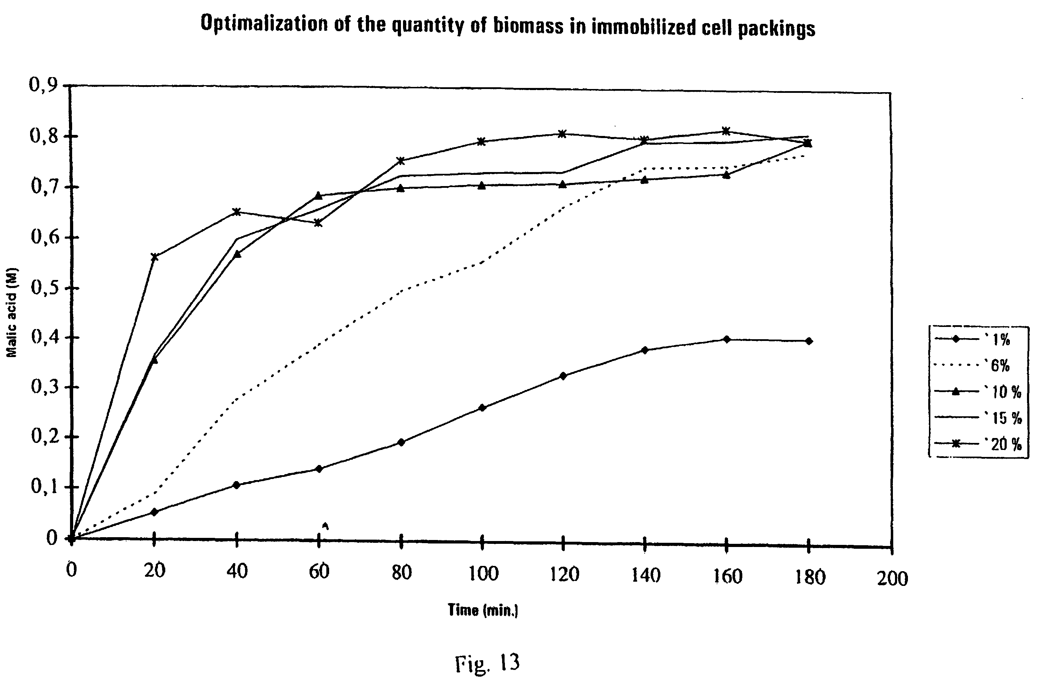

As it can be seen on Fig. 13, 5% biomass content is sufficient for the biocatalyst to reach steady state at 10 fold volume difference in 80 minutes. This means that instead of using 20% biomass content immobilized biocatalyst, as it was done in the previous experiments (and also usual in industrial biotechnology), in the case of our recombinant system, it is sufficient to use a 5% biomass content catalyst to reach optimal parameters.

Example 5

Temperature dependence of the bioreactor packed with immobilized biocatalyst comprising killed recombinant *E.coli* cells

According to the art, the fumarase C enzyme of *E.coli* is much more heat stable that other fumarases discovered earlier. It was, thus, decided to determine if the temperature optimum of the immobilized biocatalyst of the invention is shifted towards higher temperatures or not.

A bioreactor having an effective volume of 11 ml was packed with the immobilized biocatalyst according to Example 3, and the reactor was thermostated. The reactor was incubated at each temperature value for 6 days and the amount of the produced L(-) malic acid was determined each day.

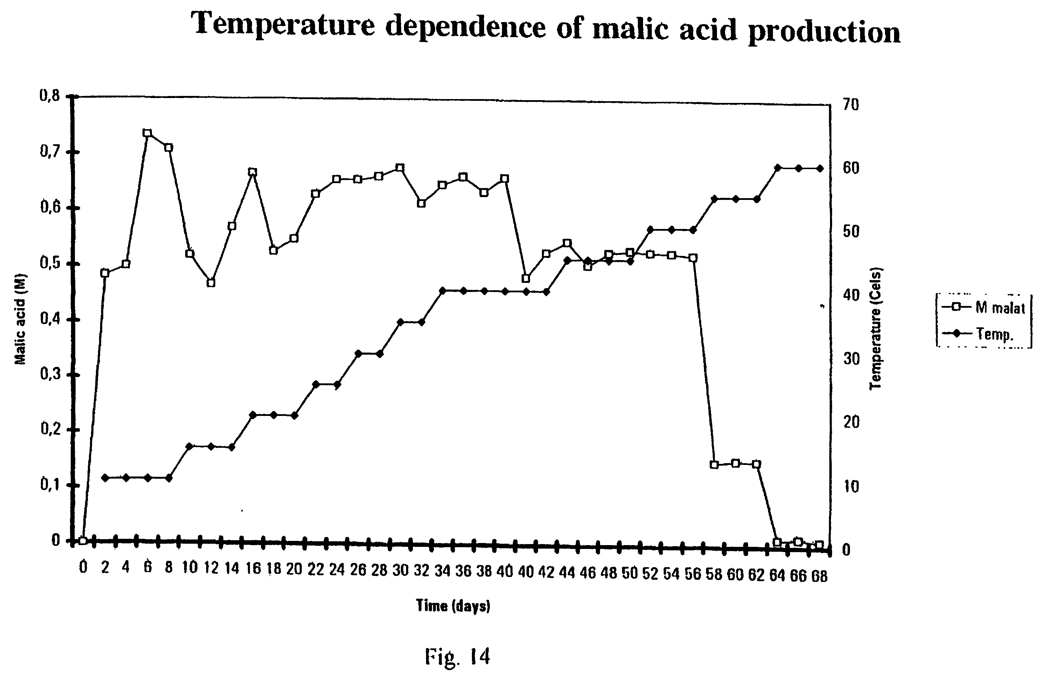

Flow rate was kept constant (at 5 ml/h) during the experiment. The results are shown on Fig. 14. The results demonstrate that the temperature optimum of the biocatalyst is about 35 °C. It can also be seen from the data shown that the thermostated system is "smoother", i.e. the measured values are not so diverse as they are in the system working at room temperature.

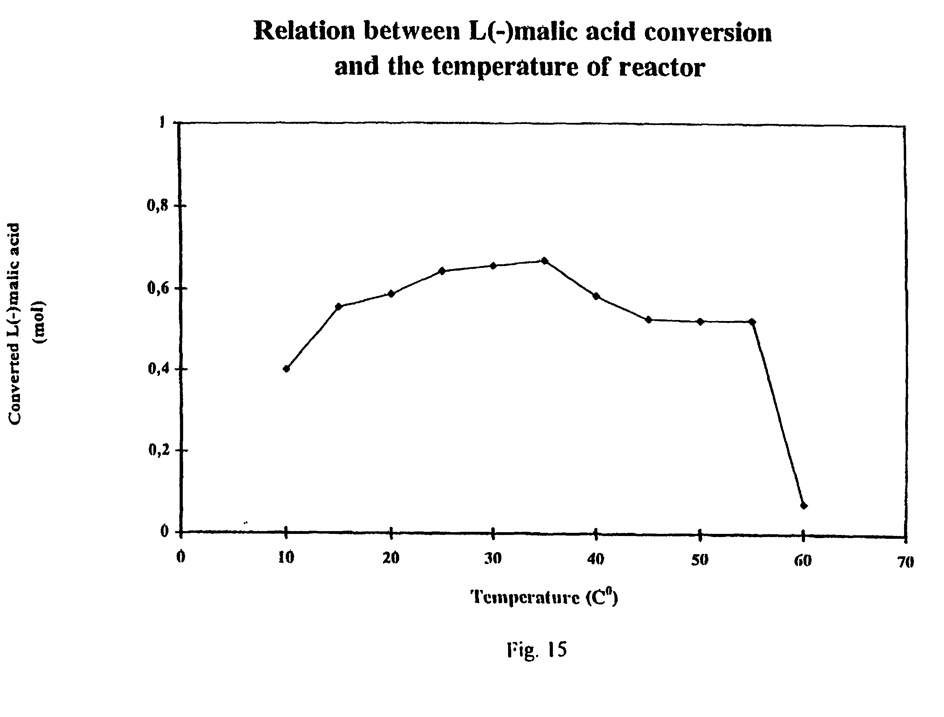

The mean conversion rate values belonging to given temperature ranges were calculated and plotted against temperature (Fig. 15). It can be established that the system has a wide optimal temperature range. This optimum value is significantly different from that was expected and also from the 37 °C value known in the art.

As it is clearly seen from the above results and disclosure, the inventors were successful in developing a novel immobilized biocatalyst having a half life significantly longer than that of traditional biocatalyst as a consequence of the new immobilization method of the invention. By employing the novel immobilized biocatalyst of the invention, a stereoselective continuous production method for L(-) malic acid production was developed that is industrially useful and significantly more effective than any previous method known from the art.

**Claims**

1. Immobilized biocatalyst comprising living cells and/or parts thereof wherein

    i) said cells and/or parts thereof are comprised in ionic strength resistant, osmotically and mechanically stable gel particles of molecular composite structure built from at least two different polyanionic substances bound together by polyvalent cations, advantageously by bivalent metal ions; one of said polyanionic substances being an ionotropic gel forming material and another of said polyanionic substances being a gel forming material having a definite gelling temperature;
    ii) optionally, a membrane of a predetermined average pore size is formed around said composite gel particles by the use of a polycationic substance; and
    iii) optionally, the cells comprised in said gel particles are killed by a treatment with a detergent and/or by sonication after being immobilized in the gel particles.

2. Immobilized biocatalyst according to claim 1, wherein said gel particles are built from alginate and a polyanionic substance of agarose related structure comprising anionic side groups, too and, optionally, the gel particles are coated by a membrane formed by using chitosan and, optionally, the immobilized cells are killed by a treatment with Na-cholate and/or by sonication.

3. Immobilized biocatalyst according to claims 1 or 2, wherein the gel particles are coated by a membrane not permeable for globular proteins of a molecular mass 50,000 D or larger.

4. Immobilized biocatalyst according to anyone of claims 1 to 3, wherein the gel particles comprise recombinant *E. coli* cells and/or parts thereof.

5. Immobilized biocatalyst according to anyone of claims 1 to 4, wherein the gel particles comprise recombinant cells - and/or parts thereof - transformed with a vector constitutively expressing fumarase C enzyme.

6. Immobilized biocatalyst according to anyone of claims 1 to 5, wherein the gel particles comprise recombinant cells - and/or parts thereof - transformed with plasmid pCKR101/FumC.

7. Immobilized biocatalyst according to anyone of claims 1 to 6, wherein the biocatalyst comprises 5-20% biomass.

8. A process for producing L(-) malic acid from fumaric acid, **characterized in** employing an immobilized biocatalyst according to anyone of claims 1 to 7 for transforming fumaric acid to L(-) malic acid and, optionally, the process is practiced in continuous production manner.

9. The process of claim 8, **characterized in** practicing said process at 35 °C.

10. The process of claims 8 or 9, **characterized in** practicing said process starting from a solution comprising at least 1 mol/l fumaric acid.

**Patentansprüche**

1. Immobilisierter Biokatalysator, enthaltend lebende Zellen oder Teile davon, in welchem

   i) die Zellen und/oder deren Teile in gegen Änderungen der Ionenstärke beständigen, osmotisch und mechanisch stabilen Gelteilchen molekularer Kompositstruktur vorliegen, die aus wenigstens zwei unterschiedlichen polyanionischen, durch mehrwertige Kationen, vorzugsweise zweiwertige Metallionen, aneinander gebundenen Substanzen aufgebaut sind, wobei die eine der polyanionischen Substanzen eine ein ionotropes Gel bildende Substanz und die andere ein gelbildender Stoff mit definierter Geliertemperatur ist;
   ii) gegebenenfalls die Gelteilchen molekularer Kompositstruktur von einer unter Verwendung von polykationischen Substanzen ausgebildeten Membran mit bestimmter durchschnittlicher Porengröße umgeben sind;
   iii) gegebenenfalls die in den Gelteilchen befindlichen Zellen nach ihrer Immobilisierung durch eine Behandlung mit Detergenzien und/oder durch Ultraschall abgetötet worden sind.

2. Immobilisierter Biokatalysator nach Anspruch 1, in dem die Gelteilchen aus Alginat und einer polyanionischen, auch anionische Seitengruppen enthaltenden Substanz agaroseähnlicher Struktur aufgebaut sind, und die Gelteilchen gegebenenfalls mit einer unter Verwendung von Chitosan ausgebildeten Membran überzogen sind, und gegebenenfalls die immobilisierten Zellen durch eine Behandlung mit Natriumcholat und/oder Ultraschall abgetötet worden sind.

3. Immobilisierter Biokatalysator nach Anspruch 1 oder 2, in dem die Gelteilchen mit einer für Globularproteine einer Molmasse $\geq$ 50 000 D undurchlässigen Membran überzogen sind.

4. Immobilisierter Biokatalysator nach einem der Ansprüche 1-3, in dem die Gelteilchen rekombinante *E.-coli*-Zellen und/oder Teile davon enthalten.

5. Immobilisierter Biokatalysator nach einem der Ansprüche 1-4, in dem die Gelteilchen mit einem konstitutiv das Enzym Fumarase C exprimierenden Vektor transformierte rekombinante Zellen und/oder Teile davon enthalten.

6. Immobilisierter Biokatalysator nach einem der Ansprüche 1-5, in dem die Gelteilchen mit dem Plasmid pCKR101/FumC transformierte rekombinante Zellen und/oder Teile davon enthalten.

7. Immobilisierter Biokatalysator nach einem der Ansprüche 1-6, enthaltend 5-20 % Biomasse.

8. Verfahren zur Herstellung von L-(-)-Äpfelsäure aus Fumarsäure, *dadurch gekennzeichnet,* **daß** zur Umwandlung der Fumarsäure in L-(-)-Äpfelsäure ein immobilisierter Biokatalysator nach einem der Ansprüche 1-7 verwendet und das Verfahren gegebenenfalls kontinuierlich ausgeführt wird.

9. Verfahren nach Anspruch 8, *dadurch gekennzeichnet,* **daß** das Verfahren bei 35 °C ausgeführt wird.

10. Verfahren nach Anspruch 8 oder 9, *dadurch gekennzeichnet,* **daß** es ausgehend von einer wenigstens 1 Mol/l Fumarsäure enthaltenden Lösung ausgeführt wird.

**Revendications**

1. Biocatalyseur immobilisé comprenant des cellules vivantes et/ou leurs parties, **caractérisé en ce que**

   i) les cellules et/ou ces parties sont immobilisées dans les particules de gel à structure moléculaire composite, stables osmotiquement et mécaniquement, résistantes aux forces ioniques, composées d'au moins deux matières polyanioniques différentes couplées par des cations polyvalents, de préférence par des ions métalliques bivalents; une des matières polyanioniques étant une matière formant un gel ionotropique et une autre matière polyanionique étant une matière formant un gel ayant une température définie de gélification;
   ii) le cas échéant, une membrane à diamètre moyen prédéterminé de pore est formée autours des particules de gel composite, par l'utilisation d'une matière polycationique; et
   iii) le cas échéant, les cellules, après avoir été immobilisées dans les particules de gel, sont tuées par un traitement par détergent et/ou par ultrason.

**2.** Biocatalyseur immobilisé selon la revendication 1, **caractérisé en ce que** les particules de gel sont formées d'alginate et d'une matière polianionique à structure similaire à l'agarose et comprenant des groupements anioniques latéraux également, et, le cas échéant, les particules de gel sont enrobées par une membrane préparée en utilisant de citosane, et, le cas échéant, les cellules immobilisées sont tuées par un traitement par colate de sodium et/ou par ultrason.

**3.** Biocatalyseur immobilisé selon la revendication 1 ou 2, **caractérisé en ce que** les particules de gel sont enrobées par une membrane non perméable aux proteines globulaires à une masse moléculaire de 50 000 D ou plus.

**4.** Biocatalyseur immobilisé selon l'une quelconque des revendications 1 à 3, **caractérisé en ce que** les particules de gel comprennent des cellules récombinantes de E.coli et/ou leurs parties.

**5.** Biocatalyseur immobilisé selon l'une quelconque des revendications 1 à 4, **caractérisé en ce que** les particules de gel comprennent des cellules récombinantes - et/ou leurs parties - transformées par un vecteur expressant constitutivement l'ensyme fumarase C.

**6.** Biocatalyseur immobilisé selon l'une quelconque des revendications 1 à 5, **caractérisé en ce que** les particules de gel comprennent des cellules récombinantes - et/ou leurs parties - transformées par plasmide pCKR101/FumC.

**7.** Biocatalyseur immobilisé selon l'une quelconque des revendications 1 à 6, **caractérisé en ce que** le biocatalyseur comprend 5 à 20 % de biomasse.

**8.** Procédé pour la préparation de L(-) acide malique à partir de l'acide fumarique, **caractérisé en ce qu'** un biocatalyseur immobilisé selon l'une quelconque des revendications 1 à 7 est utilisé pour la transformation de l'acide fumarique en L(-) acide malique, et, le cas échéant, le procédé est effectué en production continue.

**9.** Le procédé selon la revendication 8, **caractérisé en ce qu'**il est effectué à une température de 35 °C.

**10.** Le procédé selon la revendication 8 ou 9, **caractérisé en ce qu'**une solution comprenant au moins 1 mol/l de l'acide fumarique est utilisée comme produit de départ.

Fig. 1

Fig. 2

/ Guluronic acid parts (G-block)    Well arranged region

⌒ Mannuronic acid parts    ◯ Calcium 2−    Disordered region

Fig. 3

Fig. 4

——— Alginate

Gelrite

Fig. 5

# Protein permeability of membranes formed on 2 % polyanione gel as a function of polycatione concentration

Fig. 6a

EP 0 931 142 B1

# Protein permeability of membranes formed on 2 % polyanione gel as a function of polycatione concentration

Fig. 6b

EP 0 931 142 B1

7a Structure of the
alginate gel

7b Chitosan in almost
optimal quantity

7c Chitosan in optimal quantity

Fig. 7

Fig. 8

EP 0 931 142 B1

# Irregularity of optimal flow rate of bioreactor with recombinant Escherichia coli packing

Fig. 9

EP 0 931 142 B1

# Determination of optimal flow rate in the case of halophile Micrococcus roseus

Fig. 10

EP 0 931 142 B1

L(-)malic acid production characteristics

Fig. 11

Reaction time (sec)

Activity (mmol malic acid/g packing/hour)

## Malic acid production with genetically modified and immobilized bacteria

Fig. 12

EP 0 931 142 B1

29

# Optimalization of the quantity of biomass in immobilized cell packings

Fig. 13

EP 0 931 142 B1

# Temperature dependence of malic acid production

Fig. 14

EP 0 931 142 B1

# Relation between L(-)malic acid conversion and the temperature of reactor

Fig. 15

EP 0 931 142 B1